(19) **Europäisches Patentamt / European Patent Office / Office européen des brevets**

(11) **EP 4 702 930 A1**

(12) # EUROPEAN PATENT APPLICATION

(43) Date of publication:
04.03.2026 Bulletin 2026/10

(21) Application number: 25198596.6

(22) Date of filing: 28.08.2025

(51) International Patent Classification (IPC):
*A61B 6/03* (2006.01)   *A61B 6/08* (2006.01)
*A61B 6/00* (2024.01)   *A61B 6/58* (2024.01)
*A61B 34/20* (2016.01)

(52) Cooperative Patent Classification (CPC):
**A61B 6/032; A61B 6/08; A61B 6/547; A61B 6/587;**
A61B 6/4441; A61B 6/545; A61B 2034/2051;
A61M 2025/0166

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 02.09.2024 CN 202411223821

(71) Applicant: Siemens Healthineers AG
91301 Forchheim (DE)

(72) Inventors:
• **ZHANG, Yu Xuan**
**Shanghai, 200240 (CN)**
• **PENG, Xi Shuai**
**Shanghai, 200129 (CN)**
• **ZOU, Yun Zhe**
**Shanghai, 201210 (CN)**
• **ZHENG, Xiao Yuan**
**Shanghai, 201314 (CN)**
• **YUAN, Lin**
**Shanghai, 201318 (CN)**
• **MAN, YUN JIE**
**Shanghai, 201801 (CN)**

(74) Representative: HKW Intellectual Property PartG mbB
**Theresienhöhe 12**
**80339 München (DE)**

(54) **METHOD AND APPARATUS FOR ALIGNMENT OF TUBE AND DETECTOR PLATE IN X-RAY IMAGING SYSTEM, AND SYSTEM**

(57) Embodiments of the present invention disclose a method and apparatus for alignment of a tube and a detector plate in an X-ray imaging system, and a system. The method comprises: acquiring coordinates of the center of the detector plate in a magnetic field coordinate system by means of an electromagnetic tracking apparatus, wherein a magnetic field transmitter is fixed to a casing of the tube, and a magnetic sensor is fixed in a non-imaging region of the detector plate; transforming the coordinates of the center of the detector plate to a two-dimensional image coordinate system of a 3D camera; controlling the X-ray imaging system to begin exposure; during exposure, moving the tube and acquiring in real time the center of a light field of X-rays collected by the 3D camera; transforming the center of the light field of X-rays to the two-dimensional image coordinate system of the 3D camera; when the center of the light field of X-rays coincides with the center of the detector plate in the two-dimensional image coordinate system, determining that the tube and the detector plate have attained center alignment. Embodiments of the present invention increase the probability of accurate alignment of the tube and the detector plate.

Fig. 1

**Description**

TECHNICAL FIELD

The present invention relates to the technical field of medical imaging, in particular to a method and apparatus for alignment of a tube and a detector plate in an X-ray imaging system, and an X-ray imaging system.

BACKGROUND ART

In X-ray imaging, for example mobile DR (Digital Radiography) applications, correct alignment of the tube with the detector plate is of vital importance to image quality. Before exposure begins, the tube and the detector plate must be aligned, so that the emitted X-rays can be accurately projected to the imaging region of the detector plate. One challenge hindering the achievement of optical alignment is that the patient obscures the detector plate, so the technician cannot see the detector plate and is unable to unequivocally verify which region of the detector plate the X-rays emitted by the beam limiter are projected onto, and thus is unable to accurately align the tube with the detector plate.

At the present time, some solutions have already been proposed to solve this problem, the main ones being as follows:

One. A method based on an inertial navigation system. This method acquires the real-time position of the detector plate by integration using an inertia sensor. However, due to the inherent cumulative error in the integration process, such a method often results in poor positioning precision in actual applications.

Two. An RF (radio frequency) transmitter is installed on the detector plate, and an RF receiver is positioned at the tube end. Such a configuration enables distance to be measured by calculating the power loss of RF signals, and also facilitates detection of the attitude of the detector plate, wherein the configuration of the RF transmitter and RF receiver may also be replaced with an ultrasonic positioning device. However, RF transmitters and ultrasonic positioning devices typically exhibit a positioning error of more than 10 cm, which is unacceptable in actual applications.

Three. A method based on machine vision. This method monitors the movement path of the technician's hands as he places the detector behind the patient, in order to estimate the position and attitude of the detector. However, when the detector is obscured and thus not visible, the result of estimation will obviously be inaccurate.

In addition, in the process of using mobile DR to perform PICC (Peripheral Insertion of Central Catheter), precise positioning of the catheter is vital. However, the invisibility of the needle position throughout the insertion process poses a considerable challenge.

In the case of PICC, the conventional method is typically to first insert the catheter blindly, and then confirm whether the catheter insertion position is accurate based on an X-ray image of the target region. The method has a high probability of failure, and also increases discomfort for the patient and takes a long time. To improve the situation, some methods propose the use of ultrasonic guidance in conjunction with X-rays to provide real-time imaging of blood vessels, so as to confirm the catheter insertion position; however, such methods require that the technician have a high skill level, and due to the restrictions on angle of view, it is not possible to obtain a complete view of the anatomical structure of blood vessels. In addition, some methods use electromagnetic positioning in conjunction with an IC-ECG (IntraCardiac ElectroCardioGram) for PICC navigation, and while such methods have very high precision in terms of positioning and real-time tracking, they require that the electrocardiogram signals be analysed accurately to determine the needle insertion position, so place very high requirements on the technician; if the technician analyses the electrocardiogram signals incorrectly, the needle insertion position will be inaccurate.

SUMMARY OF THE INVENTION

In view of the above, embodiments of the present invention propose: a method and apparatus for alignment of a tube and a detector plate in an X-ray imaging system, so as to increase the probability of accurate alignment of the tube and the detector plate; an X-ray imaging system, to increase the probability of accurate alignment of the tube and the detector plate; and a computer program product, a computer-readable storage medium and an electronic device, to increase the probability of accurate alignment of the tube and the detector plate.

A method for alignment of a tube and a detector plate in an X-ray imaging system, the method comprising:

acquiring coordinates of the center of the detector plate in a magnetic field coordinate system by means of an electromagnetic tracking apparatus, wherein a magnetic field transmitter of the electromagnetic tracking apparatus is fixed to a casing of the tube, and a magnetic sensor of the electromagnetic tracking apparatus is fixed in a non-imaging region of the detector plate;

transforming the coordinates of the center of the detector plate in the magnetic field coordinate system to a two-dimensional image coordinate system of a 3D camera;

controlling the X-ray imaging system to begin exposure; moving the tube during exposure; acquiring, in real time during exposure, the center of a light field of X-rays collected by the 3D camera; and transforming coordinates of the center of the light field of X-rays to the two-dimensional image coordinate system of the 3D camera;

when the coordinates of the center of the light field of X-rays in the two-dimensional image coordinate system of the 3D camera coincide with the coordinates of the center of the detector plate in the two-dimensional image coordinate system of the 3D camera, determining that the tube and the detector plate have attained center alignment.

The step of transforming the coordinates of the center of the detector plate in the magnetic field coordinate system to a two-dimensional image coordinate system of a 3D camera further comprises: continuously displaying, on a display screen of the X-ray imaging system, the coordinates of the center of the detector plate in the two-dimensional image coordinate system of the 3D camera;

and the step of transforming coordinates of the center of the light field of X-rays to the two-dimensional image coordinate system of the 3D camera further comprises: displaying, in real-time on the display screen of the X-ray imaging system, the coordinates of the center of the light field of X-rays in the two-dimensional image coordinate system of the 3D camera.

The step of acquiring coordinates of the center of the detector plate in a magnetic field coordinate system by means of an electromagnetic tracking apparatus comprises:

acquiring coordinates and an attitude of the magnetic sensor in the magnetic field coordinate system; determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system.

After the step of acquiring coordinates and an attitude of the magnetic sensor in the magnetic field coordinate system, but before the step of determining coordinates of the center of the detector plate in the magnetic field coordinate system, the method further comprises:

using pre-acquired position calibration parameters and attitude calibration parameters to correct the coordinates and attitude of the magnetic sensor in the magnetic field coordinate system;

and the step of determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system, comprises: determining coordinates of the center of the detector plate in the magnetic field coordinate system, based on the corrected coordinates and attitude of the magnetic sensor in the magnetic field coordinate system, and a pre-acquired relative position and relative attitude relationship of the magnetic sensor and the center of the detector plate in the magnetic field coordinate system.

The step of acquiring coordinates of the center of the detector plate in a magnetic field coordinate system by means of an electromagnetic tracking apparatus further comprises:

acquiring an attitude of the detector plate in the magnetic field coordinate system by means of the electromagnetic tracking apparatus, and transforming the attitude of the detector plate in the magnetic field coordinate system to a tube coordinate system;

the step of acquiring, in real time during exposure, the center of a light field of X-rays collected by the 3D camera, further comprises:

acquiring an attitude of the tube in the tube coordinate system in real time during exposure, and based on the attitude of the detector plate in the tube coordinate system and the attitude of the tube in the tube coordinate system, monitoring in real time an angle between a central ray of X-rays and the plane of an imaging region of the detector plate; and when the central ray of X-rays is perpendicular to the plane of the imaging region of the detector plate, determining that the tube and the detector plate have attained angle alignment;

and when the tube and the detector plate have attained both angle alignment and center alignment, determining that the tube and the detector plate are aligned.

After the step of determining that the tube and the detector plate are aligned, the method further comprises:

before performing peripheral insertion of a central catheter, acquiring in advance an X-ray image of a target region containing a position to which a catheter must be inserted;

in the process of performing peripheral insertion of a central catheter, acquiring in real time, by means of a micro magnetic sensor fixed to a needle used for peripheral insertion of a central catheter, coordinates of the needle in the magnetic field coordinate system; transforming the coordinates of the needle in the magnetic field coordinate system to an X-ray image coordinate system in real time; based on the coordinates of the needle in the X-ray image coordinate system, displaying the position of the needle in the X-ray image in real time; and confirming whether a needle insertion position is accurate, according to the needle position displayed in real time in the X-ray image.

The step of transforming the coordinates of the needle in the magnetic field coordinate system to an X-ray image coordinate system in real time comprises:

based on a pre-acquired transformation relationship between the magnetic field coordinate system and the tube coordinate system, transforming the coordinates of the needle in the magnetic field coordinate system to the tube coordinate system in real time;
based on a distance between the tube and the detector plate in the tube coordinate system after alignment of the tube and the detector plate, and the attitude of the detector plate in the tube coordinate system, transforming the coordinates of the needle in the tube coordinate system to the X-ray image coordinate system.

The transformation relationship between the magnetic field coordinate system and the tube coordinate system is acquired in the following way:
based on a pre-acquired transformation relationship between the magnetic field coordinate system and a 3D camera coordinate system, and a transformation relationship between the 3D camera coordinate system and the tube coordinate system, the transformation relationship between the magnetic field coordinate system and the tube coordinate system is acquired.
An apparatus for alignment of a tube and a detector plate in an X-ray imaging system, the apparatus comprising:

a position acquisition module, for: acquiring coordinates of the center of the detector plate in a magnetic field coordinate system by means of an electromagnetic tracking apparatus, wherein a magnetic field transmitter of the electromagnetic tracking apparatus is fixed to a casing of the tube, and a magnetic sensor of the electromagnetic tracking apparatus is fixed in a non-imaging region of the detector plate;

a position transformation and display module, for: transforming the coordinates of the center of the detector plate in the magnetic field coordinate system to a two-dimensional image coordinate system of a 3D camera;

a monitoring and alignment module, for: controlling the X-ray imaging system to begin exposure; moving the tube during exposure; acquiring, in real time during exposure, the center of a light field of X-rays collected by the 3D camera; transforming coordinates of the center of the light field of X-rays to the two-dimensional image coordinate system of the 3D camera; and when the coordinates of the center of the light field of X-rays in the two-dimensional image coordinate system of the 3D camera coincide with the coordinates of the center of the detector plate in the two-dimensional image coordinate system of the 3D camera, determining that the tube and the detector plate have attained center alignment.

The position acquisition module acquiring coordinates of the center of the detector plate in a magnetic field coordinate system by means of an electromagnetic tracking apparatus comprises:

acquiring coordinates and an attitude of the magnetic sensor in the magnetic field coordinate system;

determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system.

The position acquisition module acquiring coordinates of the center of the detector plate in a magnetic field coordinate system by means of an electromagnetic tracking apparatus is further used for:

acquiring an attitude of the detector plate in the magnetic field coordinate system by means of the electromagnetic tracking apparatus, and transforming the attitude of the detector plate in the magnetic field coordinate system to a tube coordinate system;

the monitoring and alignment module acquiring, in real time during exposure, the center of a light field of X-rays collected by the 3D camera, is further used for:

acquiring an attitude of the tube in the tube coordinate system in real time during exposure, and based on the attitude of the detector plate in the tube coordinate system and the attitude of the tube in the tube coordinate system, monitoring in real time an angle between a central ray of X-rays and the plane of an imaging region of the detector plate; and when the central ray of X-rays is perpendicular to the plane of the imaging region of the detector plate, determining that the tube and the detector plate have attained angle alignment; and when the tube and the detector plate have attained both angle alignment and center alignment, determining that the tube and the detector plate are aligned.

The apparatus further comprises: a peripherally inserted central catheter navigation module, for:

after alignment of the tube and the detector plate, but before performing peripheral insertion of a central catheter, acquiring in advance an X-ray image of a target region containing a position to which a catheter must be inserted; in the process of performing peripheral insertion of a central catheter, acquiring in real time, by means of a micro magnetic sensor fixed to a needle used for peripheral insertion of a central catheter, coordinates of the needle in the magnetic field coordinate system;

transforming the coordinates of the needle in the magnetic field coordinate system to an X-ray image coordinate system in real time; based on the coordinates of the needle in the X-ray image coordinate system, displaying the position of the needle in the X-ray image in real time; and confirming whether a needle insertion position is accurate, according to the needle position displayed in real time in the X-ray image.

The peripherally inserted central catheter navigation module transforming the coordinates of the needle in the magnetic field coordinate system to an X-ray image coordinate system in real time comprises:

based on a pre-acquired transformation relationship between the magnetic field coordinate system and the tube coordinate system, transforming the coordinates of the needle in the magnetic field coordinate system to the tube coordinate system in real time; based on a distance between the tube and the detector plate in the tube coordinate system after alignment of the tube and the detector plate, and the attitude of the detector plate in the tube coordinate system, transforming the coordinates of the needle in the tube coordinate system to the X-ray image coordinate system.

An X-ray imaging system, comprising the apparatus as described in any one of the embodiments above.

A computer-readable storage medium, storing an instruction which, when executed by a processor, executes the steps of the method for alignment of a tube and a detector plate in an X-ray imaging system as described in any one of the embodiments above.

In embodiments of the present invention, the magnetic field transmitter is mounted on the casing of the tube and the magnetic sensor is mounted in the non-imaging region of the detector plate to form the electromagnetic tracking apparatus; then the coordinates of the center of the detector plate in the magnetic field coordinate system are acquired by means of the electromagnetic tracking apparatus, and the coordinates of the center of the detector plate in the magnetic field coordinate system are transformed to the two-dimensional image coordinate system of the 3D camera; then the X-ray imaging system is controlled to begin exposure, the tube is moved during exposure, the centre of the light field of X-rays collected by the 3D camera is acquired in real time during exposure, and coordinates of the centre of the light field of X-rays are transformed to the two-dimensional image coordinate system of the 3D camera; and when the coordinates of the center of the light field of X-rays in the two-dimensional image coordinate system of the 3D camera coincide with the coordinates of the center of the detector plate in the two-dimensional image coordinate system of the 3D camera, it is determined that the tube and the detector plate have attained center alignment. Thus, regardless of whether the detector plate is obscured, accurate center alignment of the tube and the detector plate can be achieved in any situation, increasing the probability of accurate alignment of the tube and the detector plate to the greatest extent possible; and since the positioning precision of the electromagnetic tracking apparatus is very high, the precision of tube and detector plate alignment is increased.

BRIEF DESCRIPTION OF THE DRAWINGS

Preferred embodiments of the present invention will be described in detail below with reference to the drawings, to give an ordinary person skilled in the art a clearer understanding of the abovementioned and other features and advantages of the present invention. In the drawings:

Fig. 1 is a flow chart of a method for alignment of a tube and a detector plate in an X-ray imaging system provided in an embodiment of the present invention.

Fig. 2 is a schematic drawing showing the mounting positions of two magnetic sensors on the detector plate.

Fig. 3 is a schematic drawing showing mounting positions of the EMT apparatus and the 3D camera in the X-ray imaging system.

Fig. 4 is a flow chart of a PICC navigation method provided in an embodiment of the present invention.

Fig. 5 is a structural schematic drawing of an apparatus for alignment of a tube and a detector plate in an X-ray imaging system provided in an embodiment of the present invention.

Key to the drawings:

| Label | Meaning |
| --- | --- |
| 101 - 104 | steps |
| 211, 212 | magnetic sensors |
| 22 | detector plate |
| O | center of detector plate |
| 31 | tube |
| 32 | detector plate |
| 331 | magnetic field transmitter |
| 332, 333 | magnetic sensors |
| 34 | 3D camera |
| 401 - 404 | steps |
| 50 | apparatus for alignment of tube and detector plate in X-ray imaging system |
| 51 | position acquisition module |
| 52 | position transformation and display module |
| 53 | monitoring and alignment module |

DETAILED DESCRIPTION OF EMBODIMENTS

To clarify the objective, technical solution and advantages of the present invention, the present invention is explained in further detail below by way of embodiments.

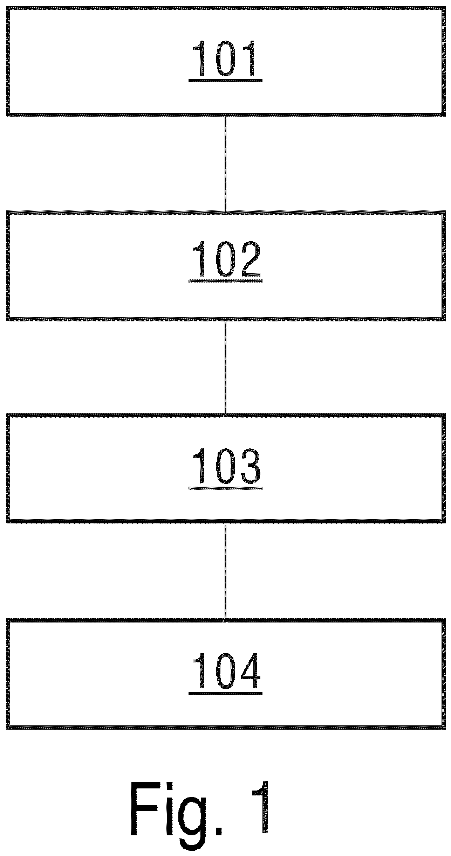

Fig. 1 is a flow chart of a method for alignment of a tube and a detector plate in an X-ray imaging system provided in an embodiment of the present invention. As shown in Fig. 1, the steps thereof are specifically as follows:

Step 101: acquiring coordinates of the center of the detector plate in a magnetic field coordinate system by means of an EMT (ElectroMagnetic Tracking) apparatus, wherein a magnetic field transmitter of the EMT apparatus is fixed to a casing of the tube, and a magnetic sensor of the EMT apparatus is fixed in a non-imaging region of the detector plate.

The magnetic field coordinate system is a coordinate system in which the EMT apparatus is located, wherein the origin of the magnetic field coordinate system is generally the position of the magnetic field transmitter; the positions and directions of three coordinate axes of the magnetic field coordinate system are not restricted, and can be defined according to preset rules.

In an optional embodiment, this step 101 specifically comprises the following steps 1011 - 1012:

Step 1011: acquiring coordinates and an attitude of the magnetic sensor in the magnetic field coordinate system.

The attitude of the magnetic sensor in the magnetic field coordinate system is: rotation matrices of the magnetic sensor relative to the three coordinate axes of the magnetic field coordinate system respectively.

Step 1012: determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system.

In an optional embodiment, after acquiring the coordinates and attitude of the magnetic sensor in the magnetic field coordinate system, step 1011 further comprises: using pre-acquired position calibration parameters and attitude calibration parameters to correct the coordinates and attitude of the magnetic sensor in the magnetic field coordinate system; and step 1012 specifically comprises: determining coordinates of the center of the detector plate in the magnetic field coordinate system, based on the corrected coordinates and attitude of the magnetic sensor in the magnetic field coordinate system, and a pre-acquired relative position and relative attitude relationship of the magnetic sensor and the center of the detector plate in the magnetic field coordinate system.

Metal objects will affect the distribution of a magnetic field, and the detector contains a large number of metal components; therefore, in the case of the magnetic sensor mounted on the detector plate, errors may occur in the position and attitude of the magnetic sensor determined on the basis of magnetic field information collected by the magnetic sensor, and in order to calibrate these errors, it is necessary to pre-acquire a position calibration matrix and an attitude calibration matrix of the magnetic sensor. The position calibration matrix and the attitude calibration matrix can be acquired in the following way:

Step 01: a magnetic sensor specially used for position and attitude calibration is placed, with a specific attitude, in a specific position in the magnetic field coordinate system, wherein the coordinates and attitude of the specific position and specific attitude in the magnetic field coordinate system are known, and set as first coordinates and a first attitude respectively; based on magnetic field information collected by the magnetic sensor, second coordinates and a second attitude of the magnetic sensor in the magnetic field coordinate system are determined, wherein the second coordinates and the second attitude are coordinates and an attitude resulting from the influence of metal.

Step 02: step 01 is repeated multiple times, so as to obtain multiple pairs of first and second coordinates, and multiple pairs of first and second attitudes; based on the multiple pairs of first and second coordinates, a position calibration matrix for calibrating each set of second coordinates to the paired set of first coordinates is calculated, and an attitude calibration matrix for calibrating each second attitude to the paired first attitude is calculated. To calculate the position calibration matrix and the attitude calibration matrix, a method such as polynomial fitting or neural network fitting may be used.

In actual applications, one or more magnetic sensors may be mounted in the non-imaging region of the detector plate; when multiple magnetic sensors are used, coordinates of the center of the detector plate in the magnetic field coordinate system can be respectively determined on the basis of the coordinates and attitude of each magnetic sensor in the magnetic field coordinate system, then the mean value of the respectively determined coordinates of the center of the detector plate in the magnetic field coordinate system can be found, and taken to be the finally used coordinates of the center of the detector plate in the magnetic field coordinate system, thus improving the precision of the finally used coordinates of the center of the detector plate in the magnetic field coordinate system.

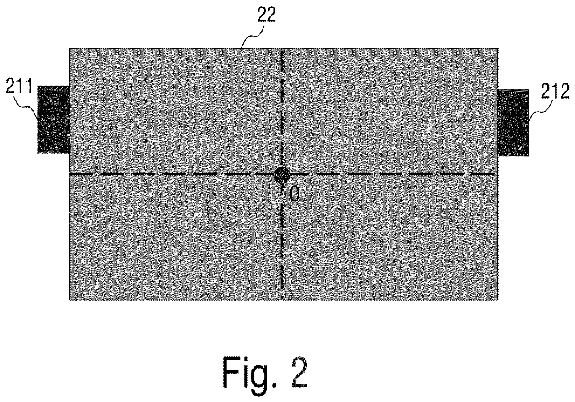

Fig. 2 is a schematic drawing showing the mounting positions of two magnetic sensors on the detector plate. As shown in Fig. 2, 211 and 212 are magnetic sensors, respectively mounted on opposite side faces (which are not in the plane of an imaging region) of the detector plate 22. The relative position relationships and relative attitude relationships of the magnetic sensors 211, 212 and the center O of the detector plate are known, so: once the corrected coordinates and attitudes of the magnetic sensors 211, 212 in the magnetic field coordinate system are known, based on the respective relative position relationships and relative attitude relationships, the coordinates of the center O of the detector plate in the magnetic field coordinate system can be respectively calculated, and the mean value of the respectively calculated coordinates of the center O of the detector plate in the magnetic field coordinate system can be found, thus obtaining the finally used coordinates of the center O of the detector plate in the magnetic field coordinate system.

Step 102: transforming the coordinates of the center of the detector plate in the magnetic field coordinate system to a two-dimensional image coordinate system of a 3D (3-dimensional) camera, and continuously displaying, on a display screen of the X-ray imaging system, the coordinates of the center of the detector plate in the two-dimensional image coordinate system of the 3D camera, wherein the 3D camera is fixed to the casing of the tube, and a mounting position of the 3D camera is such that a field of view of the 3D camera includes an imaging region of the detector plate when the tube is aligned with the detector plate. Those skilled in the art will readily understand that the mounting position of the 3D camera is not limited to being arranged on the casing of the tube, and could also be arranged at other suitable positions, e.g. other suitable positions in the X-ray imaging system or other suitable positions in an examination room containing the X-ray imaging system, such as on a roof or wall, etc.

It should be explained here that the statement that the mounting position of the 3D camera is such that the field of view of the 3D camera includes the imaging region of the detector plate when the tube is aligned with the detector plate does not require that the 3D camera be able to acquire the imaging region of the detector plate during exposure, because the 3D camera might be unable to acquire the imaging region of the detector plate during exposure if the detector is obscured by an exposure subject, etc. Here, a requirement is merely placed on the mounting position of the 3D camera, i.e. the field of view of the 3D camera must include the imaging region of the detector plate when the tube and detector plate are aligned and the detector imaging region is not obscured by anything; and the mounting position of the 3D camera is determined on the basis of this requirement.

A display screen of the 3D camera corresponds to the two-dimensional image coordinate system of the 3D camera.

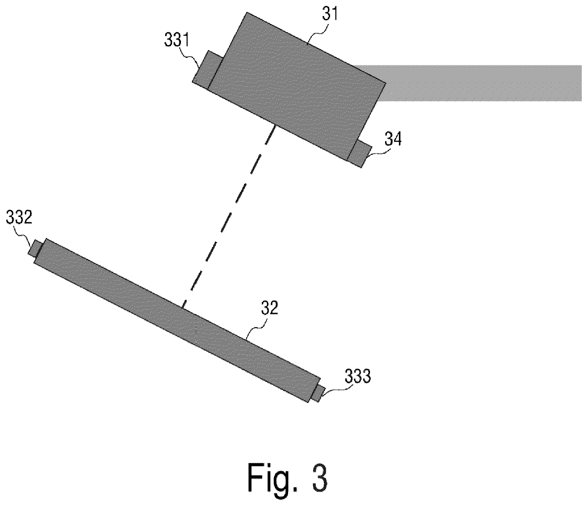

Fig. 3 is a schematic drawing showing mounting positions of the EMT apparatus and the 3D camera in the X-ray imaging system. As shown in Fig. 3, 31 is the tube, 32 is the detector plate, 331 is the magnetic field transmitter, 332 and 333 are the magnetic sensors, and 34 is the 3D camera. 331 can be fixed at any position on the casing of the tube; 332 and 333 can be fixed at any two different positions on 32 except for the imaging region; 34 is fixed to the casing of the tube, and the field of view of 34 includes the imaging region of 32 when 31 and 32 are aligned.

In an optional embodiment, transforming the coordinates of the center of the detector plate in the magnetic field coordinate system to the two-dimensional image coordinate system of the 3D camera comprises: based on pre-acquired outer parameters for transformation from the magnetic field coordinate system to a 3D camera coordinate system, transforming the coordinates of the center of the detector plate in the magnetic field coordinate system to the 3D camera coordinate system, then based on inner parameters of the 3D camera, transforming the coordinates of the center of the detector plate in the 3D camera coordinate system to the two-dimensional image coordinate system of the 3D camera.

The 3D camera coordinate system is a coordinate system in which the 3D camera is located; the origin of the 3D camera coordinate system is generally the center of the 3D camera, and the positions and directions of three coordinate axes of the 3D camera coordinate system are not restricted, and can be defined according to preset rules.

The outer parameters for transformation from the magnetic field coordinate system to the 3D camera coordinate system comprise: a rotation matrix $R_{EC}$ and a translation vector $t_{EC}$ for transformation from the magnetic field coordinate system to the 3D camera coordinate system, wherein $R_{EG}$ and $t_{EC}$ can be obtained in the following way:

A magnetic sensor specially used for transformation from the magnetic field coordinate system to the 3D camera coordinate system is designated in advance, and a visual detection sticker is stuck to the magnetic sensor; the magnetic sensor is separately placed at multiple positions, and the coordinates of these multiple positions in the magnetic field coordinate system can be obtained from magnetic field information detected by the magnetic field sensor; at the same time, the 3D camera acquires images of the magnetic sensor at the multiple positions, and detects the position of the visual detection sticker in each image, this position being the coordinates of the visual detection sticker (i.e. the magnetic sensor) in the image coordinate system of the 3D camera; next, based on the inner parameters of the 3D camera, the coordinates of the visual detection sticker in the image coordinate system of the 3D camera are transformed to the 3D camera coordinate system; thus, a coordinate pair is respectively obtained for each position: the coordinates in the magnetic field coordinate system, and the coordinates in the 3D camera coordinate system; and based on the multiple coordinate pairs, the outer parameters for transformation from the magnetic field coordinate system to the 3D camera coordinate system can be calculated.

Step 103: controlling the X-ray imaging system to begin exposure; moving the tube during exposure; acquiring, in real time during exposure, the center of a light field of X-rays collected by the 3D camera; transforming coordinates of the center of the light field of X-rays to the two-dimensional image coordinate system of the 3D camera; and displaying, in real-time on the display screen of the X-ray imaging system, the coordinates of the center of the light field of X-rays in the two-dimensional image coordinate system of the 3D camera.

After exposure begins, if there is no obstruction between the beam limiter and the detector plate, all of the X-rays will be projected onto the detector plate; otherwise, all of the X-rays will be projected onto the obstruction, or some will be projected onto the obstruction and some onto the detector plate; regardless of the manner of projection, a projection region (i.e. light field) and a surrounding region have distinguishing features, e.g. the light field will be brighter than the surrounding region, so the 3D camera will use mature technology to identify the light field and locate the center of the light field. This mature technology is not the focus of improvement in the present invention, so is not explained further here.

Step 104: when the coordinates of the center of the light field of X-rays in the two-dimensional image coordinate system of the 3D camera coincide with the coordinates of the center of the detector plate in the two-dimensional image coordinate system of the 3D camera, determining that the tube and the detector plate have attained center alignment.

In the embodiments above, the magnetic field transmitter is mounted on the casing of the tube and the magnetic sensor is mounted in the non-imaging region of the detector plate to form the EMT apparatus; then the coordinates of the center of the detector plate in the magnetic field coordinate system are acquired by means of the EMT apparatus, and the coordinates of the center of the detector plate in the magnetic field coordinate system are transformed to the two-dimensional image coordinate system of the 3D camera; then the X-ray imaging system is controlled to begin exposure, the tube is moved during exposure, the centre of the light field of X-rays collected by the 3D camera is acquired in real time during exposure, and coordinates of the centre of the light field of X-rays are transformed to the two-dimensional image coordinate system of the 3D camera; and when the coordinates of the center of the light field of X-rays in the two-dimensional image coordinate system of the 3D camera coincide with the coordinates of the center of the detector plate in the two-dimensional image coordinate system of the 3D camera, it is determined that the tube and the detector plate have attained center alignment. Thus, regardless of whether the detector plate is obscured, accurate center alignment of the tube and the detector plate can be achieved in any situation, increasing the probability of accurate alignment of the tube and the detector plate to the greatest extent possible; and since the positioning precision of the EMT apparatus is very high, the precision of tube and detector plate alignment is increased. Moreover, continuously displaying the coordinates of the center of the detector plate in the two-dimensional image coordinate system of the 3D camera on the display screen of the X-ray imaging system, and displaying in real time the coordinates of the center of the light field of X-rays in the two-dimensional image coordinate system of the 3D camera on the display screen of the X-ray imaging system, enables the technician to adjust the movement of the tube according to the position of the center of the detector plate, so that the tube and the detector plate attain center alignment as quickly as possible.

It should be explained that alignment of the tube and the detector plate comprises two aspects: one is angle alignment of the tube and the detector plate, i.e. a central ray of the X-rays emitted by the tube must be perpendicular to the plane of the imaging region of the detector plate; another is center alignment, i.e. the center of the light field of the X-rays emitted by the tube must be aligned with the center of the detector plate.

In actual applications, angle alignment and center alignment are performed synchronously. That is, step 101 further comprises: acquiring an attitude of the detector plate in the magnetic field coordinate system by means of the EMT apparatus, and transforming the attitude of the detector plate in the magnetic field coordinate system to a tube coordinate system. The attitude of the detector plate in the magnetic field coordinate system is: rotation matrices of the detector plate relative to the three coordinate axes of the magnetic field coordinate system respectively.

Moreover, step 103 further comprises: acquiring an attitude of the tube in the tube coordinate system in real time during exposure, and based on the attitude of the detector plate in the tube coordinate system and the attitude of the tube in the tube coordinate system, monitoring in real time an angle between a central ray of X-rays and the plane of the imaging region of the detector plate.

Moreover, step 104 further comprises: when the central ray of X-rays is perpendicular to the plane of the imaging region of the detector plate, determining that the tube and the detector plate have attained angle alignment.

When the tube and the detector plate have attained both angle alignment and center alignment, it is determined that the tube and the detector plate are aligned.

The tube coordinate system is a coordinate system in which the tube is located; the origin of the tube coordinate system is generally the center of the tube, and the positions and directions of three coordinate axes of the tube coordinate system are not restricted, and can be defined according to preset rules.

The tube 31 and the detector plate 32 in Fig. 3 have already attained angle alignment and center alignment.

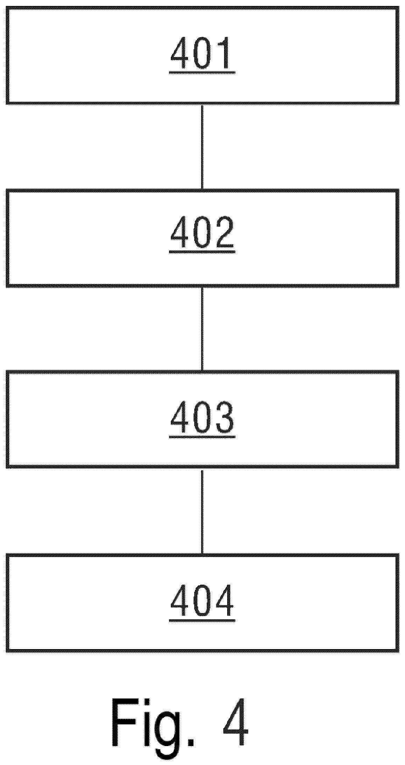

Once the tube and the detector plate are aligned, PICC can be performed. Fig. 4 is a flow chart of a PICC navigation method provided in an embodiment of the present invention. As shown in Fig. 4, the steps thereof are specifically as follows:

Step 401: an X-ray image of a target region containing a position to which a catheter must be inserted is acquired in advance.

Step 402: in the process of performing PICC, coordinates of a needle used for PICC in the magnetic field coordinate system are acquired in real time by means of a micro magnetic sensor fixed to the needle. The micro magnetic sensor is fixed to the needle.

Step 403: the coordinates of the needle in the magnetic field coordinate system are transformed in real time to an X-ray image coordinate system.

In an optional embodiment, this step 403 specifically comprises the following steps 4031 - 4032:
Step 4031: based on a pre-acquired transformation relationship between the magnetic field coordinate system and the tube coordinate system, the coordinates of the needle in the magnetic field coordinate system are transformed in real time to the tube coordinate system.

The outer parameters $R_{EC}$ and $t_{EC}$ for transformation from the magnetic field coordinate system to the 3D camera coordinate system have already been acquired in step 102; therefore, if outer parameters for transformation from the 3D camera coordinate system to the tube coordinate system are further acquired, then outer parameters for transformation from the magnetic field coordinate system to the tube coordinate system can be acquired, i.e. the transformation relationship between the magnetic field coordinate system and the tube coordinate system can be acquired. Outer parameters $R_{TC}$ (rotation matrix) and $t_{TC}$ (translation vector) for transformation from the 3D camera coordinate system to the tube coordinate system can be acquired by an existing mature technique, for example: visual detection objects are placed at multiple positions in advance, wherein the coordinates of the multiple visual detection objects in the tube coordinate system are known, and the multiple positions are all within the field of view of the 3D camera; the 3D camera acquires an image of the visual detection objects at the multiple positions, and detects the position of each visual detection object in the image, i.e. the coordinates of each visual detection object in the image coordinate system of the 3D camera; next, based on inner parameters of the 3D camera, the coordinates of each visual detection object in the image coordinate system of the 3D camera are respectively transformed to the 3D camera coordinate system; thus, a coordinate pair is respectively obtained for each position: coordinates in the tube coordinate system and coordinates in the 3D camera coordinate system; and based on the multiple coordinate pairs, the outer parameters $Rrc$ and $t_{TC}$ for transformation from the 3D camera coordinate system to the tube coordinate system can be calculated.

Then, outer parameters $R_{ET}$ (rotation matrix) and $t_{ET}$ (translation vector) for transformation from the magnetic field coordinate system to the tube coordinate system are respectively as follows:

$$R_{ET} = R_{EC}R_{TC}^{T}$$

$$t_{ET} = -R_{Ec}t_{TC} + t_{EC}$$

Letting $x_E$ denote the coordinates of the needle in the magnetic field coordinate system, the coordinates $x_T$ of the needle in the tube coordinate system are:

$$x_T = R_{ET}x_E + t_{ET}$$

Step 4032: based on a distance between the tube and the detector plate in the tube coordinate system after alignment of the tube and the detector plate, and the attitude of the detector plate in the tube coordinate system, the coordinates of the needle in the tube coordinate system are transformed to the X-ray image coordinate system.

This step 4032 is prior art and will not be further described.

Step 404: based on the coordinates of the needle in the X-ray image coordinate system, the position of the needle is displayed in real time in the X-ray image, so that: a PICC operator confirms whether a needle insertion position is accurate, according to the needle position displayed in real time in the X-ray image.

In the embodiment above, real-time, accurate PICC navigation is realized by acquiring an X-ray image of the target region in advance before performing PICC, then using the magnetic sensor fixed to the needle to track the position of the needle in real time, and transforming the coordinates of the needle to the X-ray image coordinate system.

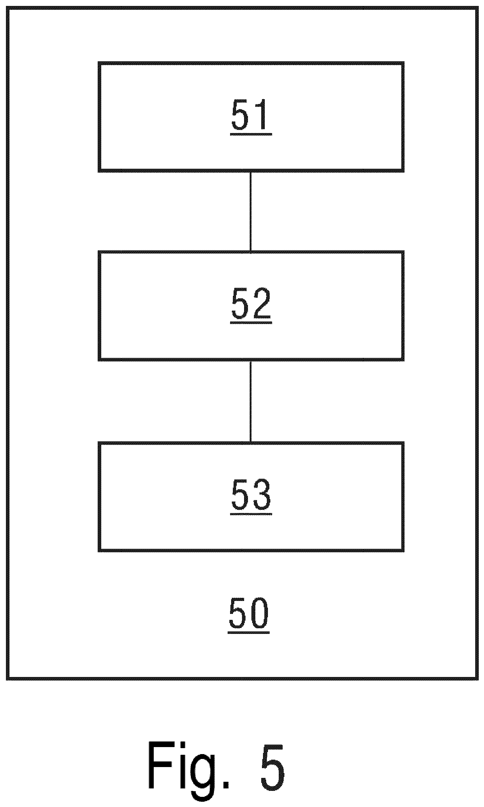

Fig. 5 is a structural schematic drawing of an apparatus 50 for alignment of a tube and a detector plate in an X-ray imaging system provided in an embodiment of the present invention. As shown in Fig. 5, the apparatus 50 mainly comprises: a position acquisition module 51, a position transformation and display module 52, and a monitoring and alignment module 53, wherein:

The position acquisition module 51 is used for: acquiring coordinates of the center of the detector plate in a magnetic field coordinate system by means of an EMT apparatus, wherein a magnetic field transmitter of the EMT apparatus is fixed to a casing of the tube, and a magnetic sensor of the EMT apparatus is fixed in a non-imaging region of the detector plate.

The position transformation and display module 52 is used for transforming the coordinates of the center of the detector plate in the magnetic field coordinate system to a two-dimensional image coordinate system of a 3D camera, wherein the 3D camera is fixed to the casing of the tube, and a mounting position of the 3D camera is such that a field of view of the 3D camera includes an imaging region of the detector plate when the tube is aligned with the detector plate.

The monitoring and alignment module 53 is used for: controlling the X-ray imaging system to begin exposure; moving the tube during exposure; acquiring, in real time during exposure, the center of a light field of X-rays collected by the 3D camera; transforming coordinates of the center of the light field of X-rays to the two-dimensional image coordinate system of the 3D camera; and when the coordinates of the center of the light field of X-rays in the two-dimensional image coordinate system of the 3D camera coincide with the coordinates of the center of the detector plate in the two-dimensional image coordinate system of the 3D camera, determining that the tube and the detector plate have attained center alignment.

In an optional embodiment, after transforming the coordinates of the center of the detector plate in the magnetic field coordinate system to the two-dimensional image coordinate system of the 3D camera, the position transformation and display module 52 is further used for: continuously displaying, on a display screen of the X-ray imaging system, the coordinates of the center of the detector plate in the two-dimensional image coordinate system of the 3D camera;

and after transforming the coordinates of the center of the light field of X-rays to the two-dimensional image coordinate system of the 3D camera, the monitoring and alignment module 53 is further used for: displaying, in real-time on the display screen of the X-ray imaging system, the coordinates of the center of the light field of X-rays in the two-dimensional image coordinate system of the 3D camera.

In an optional embodiment, the position acquisition module 51 acquiring the coordinates of the center of the detector plate in the magnetic field coordinate system by means of the EMT apparatus comprises: acquiring coordinates and an attitude of the magnetic sensor in the magnetic field coordinate system; and determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system.

In an optional embodiment, the position acquisition module 51 acquiring the coordinates of the center of the detector plate in the magnetic field coordinate system by means of the EMT apparatus is further used for: acquiring an attitude of the detector plate in the magnetic field coordinate system by means of the EMT apparatus, and transforming the attitude of the detector plate in the magnetic field coordinate system to a tube coordinate system;

and the monitoring and alignment module 53 acquiring, in real time during exposure, the center of a light field of X-rays collected by the 3D camera, is further used for: acquiring an attitude of the tube in the tube coordinate system in real time during exposure, and based on the attitude of the detector plate in the tube coordinate system and the attitude of the tube in the tube coordinate system, monitoring in real time an angle between a central ray of X-rays and the plane of the imaging region of the detector plate; and when the central ray of X-rays is perpendicular to the plane of the imaging region of the detector plate, determining that the tube and the detector plate have attained angle alignment; and when the tube and the detector plate have attained both angle alignment and center alignment, determining that the tube and the detector plate are aligned.

In an optional embodiment, the apparatus 50 further comprises: a PICC navigation module, for: after alignment of the tube and the detector plate, but before performing peripheral insertion of a central catheter (PICC), acquiring in advance an X-ray image of a target region containing a position to which a catheter must be inserted; in the process of performing PICC, acquiring in real time, by means of a micro magnetic sensor fixed to a needle used for PICC, coordinates of the needle in the magnetic field coordinate system; transforming the coordinates of the needle in the magnetic field coordinate system to an X-ray image coordinate system in real time; based on the coordinates of the needle in the X-ray image coordinate system, displaying the position of the needle in the X-ray image in real time, so that: a PICC operator confirms whether a needle insertion position is accurate, according to the needle position displayed in real time in the X-ray image.

In an optional embodiment, the PICC navigation module transforming the coordinates of the needle in the magnetic field coordinate system to the X-ray image coordinate system in real time comprises: based on a pre-acquired transformation relationship between the magnetic field coordinate system and the tube coordinate system, transforming the coordinates of the needle in the magnetic field coordinate system to the tube coordinate system in real time; and based on a distance between the tube and the detector plate in the tube coordinate system after alignment of the tube and the detector plate, and the attitude of the detector plate in the tube coordinate system, transforming the coordinates of the needle in the tube coordinate system to the X-ray image coordinate system.

Embodiments of the present invention further provide an X-ray imaging system, comprising the apparatus 50 for alignment of a tube and a detector plate in an X-ray imaging system as described in any one of the embodiments above.

It should be explained that the method and apparatus for alignment of a tube and a detector plate in an X-ray imaging system, and the X-ray imaging system provided in embodiments of the present invention may be a method, an apparatus and a system that are all applied in medical imaging.

The X-ray imaging system in embodiments of the present invention may be a mobile DR system, a non-mobile DR system, or various other X-ray imaging systems, in particular a medical imaging system. For example, when the detector plate of the X-ray imaging system is a movable component that can be removed from a detector plate fixing apparatus and used freely, the position of the detector plate can be set flexibly in multiple X-ray imaging systems used in conjunction with beds or chest radiograph stands, and the technical solution taught in the present invention can be used.

Embodiments of the present invention further provide a computer program product, comprising a computer program or instruction, which, when executed by a processor, implements the steps of the method for alignment of a tube and a detector plate in an X-ray imaging system as described in any one of the above embodiments.

Embodiments of the present invention further provide a computer-readable storage medium, storing an instruction which, when executed by a processor, can execute the steps of the method for alignment of a tube and a detector plate in an X-ray imaging system as described above. In practical applications, the computer-readable medium may be included in each device/apparatus/system in the above embodiments, or may exist independently without being fitted into the device/apparatus/system. The computer-readable storage medium stores an instruction which, when executed by a processor, can execute the steps of the method for alignment of a tube and a detector plate in an X-ray imaging system as described above.

Embodiments of the present invention further provide an electronic device. The electronic device may include a processor with one or more processing cores, a memory with one or more computer-readable storage media, and a computer program stored in the memory and executable on the processor. When the program in the memory is executed, the method described above for alignment of a tube and a detector plate in an X-ray imaging system can be implemented.

Those skilled in the art will understand that features stated in the various embodiments and/or claims disclosed in the present application can be combined and/or integrated in various ways, even if such combinations or integrations are not clearly stated in the present application. In particular, without departing from the spirit and teaching of the present application, features stated in the various embodiments and/or claims of the present application can be combined and/or integrated in various ways, and all such combinations and/or integrations fall within the scope of disclosure of the present application.

Specific embodiments have been used herein to expound the principles and forms of implementation of the present application, but the description of the embodiments above is merely intended to help understand the method of the present application and the core idea thereof, not to restrict the present application. Those skilled in the art may change the specific manner of implementation and application scope, based on the idea, spirit and principles of the present application, and any modifications, equivalent replacements, improvements, etc. made by those skilled in the art should be included within the scope of protection of the present application.

**Claims**

1. A method for alignment of a tube and a detector plate in an X-ray imaging system, **characterized in that** the method comprises:

   acquiring coordinates of the center of the detector plate in a magnetic field coordinate system by means of an electromagnetic tracking apparatus, wherein a magnetic field transmitter of the electromagnetic tracking apparatus is fixed to a casing of the tube, and a magnetic sensor of the electromagnetic tracking apparatus is fixed in a non-imaging region of the detector plate;
   transforming the coordinates of the center of the detector plate in the magnetic field coordinate system to a two-dimensional image coordinate system of a 3D camera;
   controlling the X-ray imaging system to begin exposure; moving the tube during exposure; acquiring, in real time during exposure, the center of a light field of X-rays collected by the 3D camera; and transforming coordinates of the center of the light field of X-rays to the two-dimensional image coordinate system of the 3D camera;
   when the coordinates of the center of the light field of X-rays in the two-dimensional image coordinate system of the 3D camera coincide with the coordinates of the center of the detector plate in the two-dimensional image coordinate system of the 3D camera, determining that the tube and the detector plate have attained center alignment.

2. The method as claimed in claim 1, **characterized in that** the step of transforming the coordinates of the center of the detector plate in the magnetic field coordinate system to a two-dimensional image coordinate system of a 3D camera further comprises: continuously displaying, on a display screen of the X-ray imaging system, the coordinates of the center of the detector plate in the two-dimensional image coordinate system of the 3D camera;
   and the step of transforming coordinates of the center of the light field of X-rays to the two-dimensional image coordinate system of the 3D camera further comprises: displaying, in real-time on the display screen of the X-ray imaging system, the coordinates of the center of the light field of X-rays in the two-dimensional image coordinate system of the 3D camera.

3. The method as claimed in claim 1, **characterized in that** the step of acquiring coordinates of the center of the detector plate in a magnetic field coordinate system by means of an electromagnetic tracking apparatus comprises:

   acquiring coordinates and an attitude of the magnetic sensor in the magnetic field coordinate system;
   determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system.

4. The method as claimed in claim 3, **characterized in that** after the step of acquiring coordinates and an attitude of the magnetic sensor in the magnetic field coordinate system, but before the step of determining coordinates of the center of the detector plate in the magnetic field coordinate system, the method further comprises:

using pre-acquired position calibration parameters and attitude calibration parameters to correct the coordinates and attitude of the magnetic sensor in the magnetic field coordinate system;

and the step of determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system, comprises:

determining coordinates of the center of the detector plate in the magnetic field coordinate system, based on the corrected coordinates and attitude of the magnetic sensor in the magnetic field coordinate system, and a pre-acquired relative position and relative attitude relationship of the magnetic sensor and the center of the detector plate in the magnetic field coordinate system.

5. The method as claimed in claim 1, **characterized in that** the step of acquiring coordinates of the center of the detector plate in a magnetic field coordinate system by means of an electromagnetic tracking apparatus further comprises:

acquiring an attitude of the detector plate in the magnetic field coordinate system by means of the electromagnetic tracking apparatus, and transforming the attitude of the detector plate in the magnetic field coordinate system to a tube coordinate system;

the step of acquiring, in real time during exposure, the center of a light field of X-rays collected by the 3D camera, further comprises:

acquiring an attitude of the tube in the tube coordinate system in real time during exposure, and based on the attitude of the detector plate in the tube coordinate system and the attitude of the tube in the tube coordinate system, monitoring in real time an angle between a central ray of X-rays and the plane of an imaging region of the detector plate; and when the central ray of X-rays is perpendicular to the plane of the imaging region of the detector plate, determining that the tube and the detector plate have attained angle alignment;

and when the tube and the detector plate have attained both angle alignment and center alignment, determining that the tube and the detector plate are aligned.

6. The method as claimed in claim 5, **characterized in that** after the step of determining that the tube and the detector plate are aligned, the method further comprises:

before performing peripheral insertion of a central catheter, acquiring in advance an X-ray image of a target region containing a position to which a catheter must be inserted;

in the process of performing peripheral insertion of a central catheter, acquiring in real time, by means of a micro magnetic sensor fixed to a needle used for peripheral insertion of a central catheter, coordinates of the needle in the magnetic field coordinate system; transforming the coordinates of the needle in the magnetic field coordinate system to an X-ray image coordinate system in real time; based on the coordinates of the needle in the X-ray image coordinate system, displaying the position of the needle in the X-ray image in real time; and confirming whether a needle insertion position is accurate, according to the needle position displayed in real time in the X-ray image.

7. The method as claimed in claim 6, **characterized in that** the step of transforming the coordinates of the needle in the magnetic field coordinate system to an X-ray image coordinate system in real time comprises:

based on a pre-acquired transformation relationship between the magnetic field coordinate system and the tube coordinate system, transforming the coordinates of the needle in the magnetic field coordinate system to the tube coordinate system in real time;

based on a distance between the tube and the detector plate in the tube coordinate system after alignment of the tube and the detector plate, and the attitude of the detector plate in the tube coordinate system, transforming the coordinates of the needle in the tube coordinate system to the X-ray image coordinate system.

8. The method as claimed in claim 7, **characterized in that** the transformation relationship between the magnetic field coordinate system and the tube coordinate system is acquired in the following way:

based on a pre-acquired transformation relationship between the magnetic field coordinate system and a 3D camera coordinate system, and a transformation relationship between the 3D camera coordinate system and the tube coordinate system, the transformation relationship between the magnetic field coordinate system and the tube coordinate system is acquired.

9. An apparatus for alignment of a tube and a detector plate in an X-ray imaging system, **characterized in that** the apparatus comprises:

a position acquisition module, for: acquiring coordinates of the center of the detector plate in a magnetic field coordinate system by means of an electromagnetic tracking apparatus, wherein a magnetic field transmitter of the electromagnetic tracking apparatus is fixed to a casing of the tube, and a magnetic sensor of the electromagnetic tracking apparatus is fixed in a non-imaging region of the detector plate;

a position transformation and display module, for: transforming the coordinates of the center of the detector plate in the magnetic field coordinate system to a two-dimensional image coordinate system of a 3D camera;

a monitoring and alignment module, for: controlling the X-ray imaging system to begin exposure; moving the tube during exposure; acquiring, in real time during exposure, the center of a light field of X-rays collected by the 3D camera; transforming coordinates of the center of the light field of X-rays to the two-dimensional image coordinate system of the 3D camera; and when the coordinates of the center of the light field of X-rays in the two-dimensional image coordinate system of the 3D camera coincide with the coordinates of the center of the detector plate in the two-dimensional image coordinate system of the 3D camera, determining that the tube and the detector plate have attained center alignment.

10. The apparatus as claimed in claim 9, **characterized in that** the position acquisition module acquiring coordinates of the center of the detector plate in a magnetic field coordinate system by means of an electromagnetic tracking apparatus comprises:

acquiring coordinates and an attitude of the magnetic sensor in the magnetic field coordinate system;

determining coordinates of the center of the detector plate in the magnetic field coordinate system, based on the coordinates and attitude of the magnetic sensor in the magnetic field coordinate system, and a pre-acquired relative position and relative attitude relationship of the magnetic sensor and the center of the detector plate in the magnetic field coordinate system.

11. The apparatus as claimed in claim 9, **characterized in that** the position acquisition module acquiring coordinates of the center of the detector plate in a magnetic field coordinate system by means of an electromagnetic tracking apparatus is further used for:

acquiring an attitude of the detector plate in the magnetic field coordinate system by means of the electromagnetic tracking apparatus, and transforming the attitude of the detector plate in the magnetic field coordinate system to a tube coordinate system;

the monitoring and alignment module acquiring, in real time during exposure, the center of a light field of X-rays collected by the 3D camera, is further used for:

acquiring an attitude of the tube in the tube coordinate system in real time during exposure, and based on the attitude of the detector plate in the tube coordinate system and the attitude of the tube in the tube coordinate system, monitoring in real time an angle between a central ray of X-rays and the plane of an imaging region of the detector plate; and when the central ray of X-rays is perpendicular to the plane of the imaging region of the detector plate, determining that the tube and the detector plate have attained angle alignment; and when the tube and the detector plate have attained both angle alignment and center alignment, determining that the tube and the detector plate are aligned.

12. The apparatus as claimed in claim 11, **characterized in that** the apparatus further comprises: a peripherally inserted central catheter navigation module, for:
after alignment of the tube and the detector plate, but before performing peripheral insertion of a central catheter, acquiring in advance an X-ray image of a target region containing a position to which a catheter must be inserted; in the process of performing peripheral insertion of a central catheter, acquiring in real time, by means of a micro magnetic sensor fixed to a needle used for peripheral insertion of a central catheter, coordinates of the needle in the magnetic field coordinate system; transforming the coordinates of the needle in the magnetic field coordinate system to an X-ray image coordinate system in real time; based on the coordinates of the needle in the X-ray image coordinate system, displaying the position of the needle in the X-ray image in real time; and confirming whether a needle insertion position is accurate, according to the needle position displayed in real time in the X-ray image.

13. The apparatus as claimed in claim 12, **characterized in that** the peripherally inserted central catheter navigation module transforming the coordinates of the needle in the magnetic field coordinate system to an X-ray image coordinate system in real time comprises:

based on a pre-acquired transformation relationship between the magnetic field coordinate system and the tube coordinate system, transforming the coordinates of the needle in the magnetic field coordinate system to the tube coordinate system in real time; based on a distance between the tube and the detector plate in the tube coordinate system after alignment of the tube and the detector plate, and the attitude of the detector plate in the tube coordinate system, transforming the coordinates of the needle in the tube coordinate system to the X-ray image coordinate system.

14. An X-ray imaging system, **characterized in that** the system comprises the apparatus as claimed in any one of claims 9 - 13.

15. A computer-readable storage medium, **characterized in that** the computer-readable storage medium stores an instruction which, when executed by a processor, executes the steps of the method for alignment of a tube and a detector plate in an X-ray imaging system as claimed in any one of claims 1 - 8.

Fig. 1

Fig. 2

Fig. 3

Fig. 4

Fig. 5

Europäisches
Patentamt

European
Patent Office

Office européen
des brevets

# EUROPEAN SEARCH REPORT

**Application Number**

EP 25 19 8596

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DE 10 2013 219137 A1 (SIEMENS AG [DE]) 26 March 2015 (2015-03-26) * paragraph [0001] * * paragraph [0027] - paragraph [0032] * * claims 1-10 * * figure 1 * ----- | 1-15 | INV. A61B6/03 A61B6/08 A61B6/00 A61B6/58 ADD. A61B34/20 |
| Y | US 2019/021690 A1 (MUNIER BERNARD [FR] ET AL) 24 January 2019 (2019-01-24) * paragraph [0006] - paragraph [0010] * * paragraph [0044] - paragraph [0048] * * paragraph [0062] - paragraph [0069] * * paragraph [0096] * * figures 1,4 * * claims 7-11 * ----- | 1-15 | |
| Y | US 2009/290771 A1 (FRANK KEVIN J [US] ET AL) 26 November 2009 (2009-11-26) * paragraph [0037] - paragraph [0042] * * paragraph [0046] - paragraph [0077] * * figures 1,3 * ----- | 1,3-15 | |
| A | | 2 | |
| A | US 2022/031400 A1 (HOD URIEL [IL] ET AL) 3 February 2022 (2022-02-03) * figures 1-4 * * paragraph [0001] - paragraph [0056] * * claims 1-20 * ----- | 1-15 | TECHNICAL FIELDS SEARCHED (IPC) A61B |
| A | EP 4 316 376 A1 (KONINKLIJKE PHILIPS NV [NL]) 7 February 2024 (2024-02-07) * paragraph [0056] - paragraph [0075] * * figures 1-5 * * claim 14 * ----- | 1-15 | |

The present search report has been drawn up for all claims

| Place of search | Date of completion of the search | Examiner |
|---|---|---|
| Munich | 19 January 2026 | De la Hera, Germán |

## ANNEX TO THE EUROPEAN SEARCH REPORT
## ON EUROPEAN PATENT APPLICATION NO.

EP 25 19 8596

This annex lists the patent family members relating to the patent documents cited in  the above-mentioned European search report.
The members are as contained in the European Patent Office EDP file on
The European Patent Office is in no way liable for these particulars which are merely given for the purpose of information.

19-01-2026

| Patent document cited in search report | Publication date | Patent family member(s) | | Publication date |
|---|---|---|---|---|
| DE 102013219137 A1 | 26-03-2015 | NONE | | |
| US 2019021690 A1 | 24-01-2019 | CN | 108463171 A | 28-08-2018 |
| | | EP | 3380016 A1 | 03-10-2018 |
| | | FR | 3044200 A1 | 26-05-2017 |
| | | JP | 2018535020 A | 29-11-2018 |
| | | KR | 20180084132 A | 24-07-2018 |
| | | US | 2019021690 A1 | 24-01-2019 |
| | | WO | 2017089403 A1 | 01-06-2017 |
| US 2009290771 A1 | 26-11-2009 | US | 2004215071 A1 | 28-10-2004 |
| | | US | 2009290771 A1 | 26-11-2009 |
| | | US | 2012027261 A1 | 02-02-2012 |
| US 2022031400 A1 | 03-02-2022 | CN | 114052904 A | 18-02-2022 |
| | | EP | 3944835 A1 | 02-02-2022 |
| | | IL | 285191 A | 01-02-2022 |
| | | JP | 7776053 B2 | 26-11-2025 |
| | | JP | 2022027695 A | 14-02-2022 |
| | | US | 2022031400 A1 | 03-02-2022 |
| EP 4316376 A1 | 07-02-2024 | CN | 119604239 A | 11-03-2025 |
| | | EP | 4316376 A1 | 07-02-2024 |
| | | EP | 4536085 A1 | 16-04-2025 |
| | | JP | 2025528031 A | 26-08-2025 |
| | | US | 2025255572 A1 | 14-08-2025 |
| | | WO | 2024027969 A1 | 08-02-2024 |

EPO FORM P0459

For more details about this annex : see Official Journal of the European Patent Office, No. 12/82